# NEW EUROPEAN PATENT SPECIFICATION

(11) **EP 1 575 377 B2**
(45) Date of publication and mention of the opposition decision: **21.01.2015**
(45) Mention of the grant of the patent: 15.04.2009
(21) Application number: 03795909.5
(22) Date of filing: 17.12.2003
(51) Int. Cl.: A23L 1/30, A23L 1/29

(54) **METHOD OF PREPARING A NUTRITIONAL FORMULA COMPRISING L(+) LACTIC ACID**
VERFAHREN ZUR HERSTELLUNG EINER NAHRUNGSZUSAMMENSETZUNG ENTHALTEND L(+)-MILCHSÄURE
PROCÉDÉ DE PRÉPARATION D'UNE COMPOSITION NUTRITIONELLE COMPRENANT DE L'ACIDE LACTIQUE L(+)

(30) Priority: 17.12.2002 EP 02028285
(43) Date of publication of application: 21.09.2005
(73) Proprietor: Nestec S.A., 1800 Vevey (CH)
(72) Inventor: PETERMANN, Robert, CH-3507 Biglen (CH); JOOSTEN, Henricus, CH-1074 Mollie-Margot (CH)
(74) Representative: Plougmann & Vingtoft A/S
(86) International application number: PCT/EP2003/014400
(87) International publication number: WO 2004/054371

(56) References cited:
- WO-A-96/31130
- US-A- 4 303 692
- DATABASE BIOSIS [Online] BIOSCIENCES INFORMATION SERVICE, PHILADELPHIA, PA, US; 1989, BRUNSER O ET AL: "EFFECT OF AN ACIDIFIED MILK ON DIARRHEA AND THE CARRIER STATE IN INFANTS OF LOW SOCIO-ECONOMIC STRATUM" XP002230476 Database accession no. PREV198988041353 & ACTA PAEDIATRICA SCANDINAVICA, vol. 78, no. 2, 1989, pages 259-264, ISSN: 0001-656X
- ANONYMOUS: "17th International Congress of Nutrition" INTERNET ARTICLE, [Online] XP002230474 Retrieved from the Internet: URL:http://www.univie.ac.at/iuns2001/sw_3. htm> [retrieved on 2003-02-10]
- ANONYMOUS: "Lactobacillus" INTERNET ARTICLE, [Online] XP002230475 Retrieved from the Internet: URL:http://www.flora-balance.com/lactic_ac id_producing_bacteria.htm> [retrieved on 2003-02-10]

## Description

### Chemically Acidified Formula

The present invention relates to a process of manufacturing a nutritional formula.

### The Background Art

Gastrointestinal infections, leading to diarrhoea, are still one of the principle causes of death during infancy. Bacterial infections, caused by the pathogen or its toxins, are mainly due to bacterial contamination of water or food.

Formula feeding may increase risks for gastrointestinal infections if contamination cannot be substantially excluded or if external factors favour growth of infective agents, such as warm humid seasons in tropical or subtropical countries. Typical bacterial pathogens are enteropathogentic and enterotoxigenic strains of *Escherichia coli, Salmonella, Shigella,* for example. However, viruses (rotaviruses, caliciviruses) and protozoic parasites, such as *Cryptosporidium* are also frequently associated with infant diarrhoea.

Breast-feeding reduces the exposure to these pathogens and also supplies the infant with protective antibodies against food-borne pathogens, resulting in lower incidence of diarrhoea. However, breast-feeding is not always possible, for example if a mother has to leave her child at home or with childcare during work. Furthermore, breast-feeding is not recommended if there is a risk of HIV transmission (Weinberg G. The dilemma of postnatal mother-to-child transmission of HIV: to breastfeed or not? Birth 2000; 27(3).

One way of solving the problem is the addition of a specific anti-microbial agent, as is taught in WO 96/25054. However, for infants the consumption of anti-microbial agents on a regular base should be avoided because of potential damage to the liver and, in addition because anti-microbial agents often exhibit undesirable side effects.

A nutritionally safe and effective way of inhibiting growth of bacteria in a reconstituted infant formula is acidification. Under the trademarks Pelargon®, Bionan® and Bioguigoz® (SOCIETEE DES PRODUITS NESTLE) powdered infant formulas are commercialised that have, upon reconstitution, a relatively low pH, thus reducing the risk of gastrointestinal infections.

However, the process through which acidification is achieved for these formulas is time and cost intensive: the basic ingredients of an infant formula are fermented with lactic acid bacteria until a specific PH is achieved, the fermentation is interrupted, the liquid is pasteurised and processed to a powder. The fermentation has to be controlled carefully, because it may provide growth possibilities for pathogenic bacteria and also for bacteriophages, which can interfere with the fermentation process.

Furthermore, the pH of the formulas of the state of the art can hardly be adjusted very accurately with respect to the final product and cannot be standardised on a very specific value.

However time-consuming the fermentation of nutritional formulas may be, it has the benefit that the acidification occurs slowly and steadily over a prolonged time, which is an advantage as far as the acidification of the product itself is concerned. A quick acidification of complex compositions such as nutritional formulas, which comprise a range of completely different components (proteins, carbohydrates, lipids) is delicate and often results in precipitation of certain components, phase separation or simply unsatisfactory formulas.

It is therefore an object of the present invention to provide a method of preparing a nutritional formula with a bacteriostatic activity which can be prepared more economically (without fermentation), is nutritionally safe and does not comprise antimicrobial agents other than lactic acid.

### Summary of the Invention

Remarkably, a way was found to prepare nutritional formulas based on direct acidification with L(+)-lactic acid, having bacteriostatic activity and being nutritionally safe for infants.

Consequently, in a first aspect the present invention provides a method according to claim 1.

An advantage of the present invention is that the formula prepared according to claim 1 shows bacteriostatic activity and a fermentation step may be avoided.

Yet another advantage of the present invention is that it provides an acidified nutritional formula that can be produced quickly.

### Detailed Description of the Invention

Within the context of this specification the word "comprises" is taken to mean "includes, among other things". It is not intended to be construed as "consists of only".

Within the context of the present invention L(+)-lactic acid is understood as an equivalent of S(+)-2-hydroxy propionic acid.

The term "directly acidified" refers to the fact that acid is directly added to the nutritional formula during its preparation. The acidity of L(+)-lactic acid is not obtained by a fermentation process of the formula, wherein lactic acid bacteria produce the lactic acid and the formula is thus continuously acidified over a time span of usually 2-10 hours.

The term "protein source" is not intended to be construed as including only proteins, but includes any matter that is proteinogenic, that is, may be ustilized by the human or animal body to synthesize proteins. Examples, besides intact proteins, are hydrolysed proteins and free amino acids.

All percentages are percentages by weight, unless otherwise indicated.

The fact that the formula is directly acidified may easily be determined with analytical methods detecting the presence of live or dead lactic acid bacteria or the presence of metabolites of such acidifying bacteria.

For example, quantitative DNA analysis may be suitable to determine if a formula has been fermented by a lactic acid bacterium or directly acidified. Species-specific DNA probes of lactic acid bacteria have been described in the scientific literature and may easily be conceived and used by the skilled person.

If the nutritional formula comprises bacterial DNA of a fermenting bacterium in amounts higher to the DNA corresponding to 10⁵ bacteria/g formula (dry matter), the formula has been fermented.

Other methods for determining if a nutritional formula has been subjected to a fermentation process may be selected depending on the remaining ingredients of the nutritional formula. For example, individual free amino acids, small peptides and/or non-protein nitrogen may be determined if the formula is based on intact protein, because the activity of fermenting lactic acid bacteria changes the concentration of these compounds.

Free amino-acids, peptides and other metabolites are usually specific for the fermenting activity or metabolism of a specific strain of fermenting bacteria and the presence of these metabolites is to be analysed after having determined, by DNA analysis, for example, which lactic acid bacteria has fermented the formula.

For a powdered formula, "in its liquid state" refers to the liquid, hydrated or reconstituted nutritional formula obtained usually by adding a predetermined amount of water to a predetermined amount of powdered formula. Usually, the amounts of water and/or powder are specific for a particular powdered formula.

With respect to total lactic acid of the nutritional formula, L(+)-lactic acid is predominantly present, meaning that at least 70%, preferably at least 80%, more preferably at least 90%, even more preferably at least 95% by weight of lactic acid is present in the L(+) form. For example, 96%, 97%, 98% or 99% of total lactic acid is present in the form of L(+)-lactic acid.

The term "isolated" or "purified" L(+)-lactic acid refers to lactic acid wherein the L(+) enantiomer is present, in relation to the other enantiomer of lactic acid, in the weight percentages given above.

Within the context of the present invention, the term "reconstitutable" formula refers to the fact that a formula is present basically in the form of a powder and may be prepared ad hoc by addition of a defined amount of liquid, such as water and optional stirring, for example.

Within the context of the present invention, the term "solution" in the context of the manufacturing of the present invention, is not intended to be construed as liquids were all ingredients are completely dissolved. To the contrary, for the sake of convenience the term solution is also used when actually an emulsion and/or a dispersion is present.

The pH of the formula prepared according to the invention, optionally after reconstitution with water, is in the range of 3.5 to 6, preferably 3.5 - 5.5, more preferably in the range of 4.0 to 5.3, even more preferably in the range of 4.5 to 5.0, such as 4.6 to 4.8, for example.

The nutritional formula prepared according to the invention is a powdered nutritional formula. For example, it is a powdered and reconstitutable formula.

As the nutritional composition prepared according to the invention is powdered, 100-150 g, preferably 120-140 g of the powdered may preferably be reconstituted with 900 ml water.

As powdered composition is prepared according to the present invention, there is a specific advantage as compared to the preparation of biologically acidified compositions: Since a fermentation step is avoided, the optional drying process will be much more efficient, due to the fact that a fermentable solution with low dry-matter content may be avoided. The whole process may be conducted at higher dry matter, thus superseding evaporation-step or drying of a solution at a high water content.

In an embodiment, the nutritional formula prepared according to the present invention is an infant formula.

The protein source comprises a protein source selected from the group of whole or skimmed milk powder, casein, whey protein, soy protein, rice protein, carob seed protein, germ flour protein, and/or mixtures thereof.

In still another embodiment of the nutritional formula prepared according to the invention are according to the embodiment wherein the protein source comprises whey and casein, the casein and/or whey protein is intact or not hydrolysed.

However, the formula prepared according to the invention may also comprise hydrolised protein sources, such as partially or totally hydrolysed whey and/or casein, or vegetal protein sources, for example.

The method according to the present invention comprises the step of adding a lipid source. The step of adding a lipid source is conducted before adding L-(+) lactic acid.

Preferably, the formula prepared according to the invention does not comprise remainders, waste or residues of bacteria used to acidify the nutritional formula. The formula may, in contrast, comprise living probiotics, such as encapsulated, spray dried probiotic microorganisms that may be added in powder form to a powdered nutritional formula.

Ingredients suitable for nutritional formulas may be selected from a number of different sources.

A protein source, for example, may be selected from animal and/or vegetal origin. For example, proteins from legumes, such as soy-protein, cereals, meat, or milk may be used. For example, whole milk and/or skimmed milk and/or their respective powders may be selected. Preferably, the protein source comprises whey and/or casein. Casein may be in the form of a salt (sodium or potassium caseinate), micellar, enzymatically hydrolysed or otherwise processed casein. Whey protein may be sweet or acid whey and may be hydrolysed or not. If sweet whey is used, it may be treated to the end that the CGMP fraction is removed (see for example, EP 0880 902). Hydrolysis may be conducted enzymatically or by aid of acid. It may be a complete or a partial hydrolysis, yielding poly-, oligopeptides or free amino acids. Preferably, non-hydrolysed casein and/or whey protein is used to prepare the formula.

If milk protein is used as a protein source, the milk protein may be casein, whey, or further purified fractions, such as lactalbumin, for example. Preferably the nutritional formula prepared according to the invention comprises, in percent by weight of the total protein source, 30-70%, preferably 40-60% whey and 70-30%, preferably 60-40% casein, more preferably 45-55% whey and 55-45% casein.

For example, the formula prepared according to the invention may be cow's milk based with an unchanged ratio of caseins to whey proteins. In this embodiment, the formula is a casein predominant formula. In an alternative embodiment, the may have a modified ratio of caseins and whey proteins, that is, an adapted formula, in which whey protein is in equal proportion with casein or predominant over casein. Alternatively, the formula may be an "all-whey" formula, in which whey protein may be the exclusive protein source, for example.

The protein source may provide 8-20% of the energy of the nutritional formula. Preferably, the protein source provides 9-17%, more preferably 10-15%, for example 12% of the energy of the formula.

The carbohydrate source in the nutritional formula prepared according to the invention can be carbohydrate suitable for use in infant formulas, if an infant formula is to be prepared. Typical carbohydrate sources include sucrose, maltodextrin, maltose, lactose, corn syrup, corn syrup solids, rice syrup solids, starches, and the like. Preferably, the carbohydrate source includes lactose and starch or a derivative thereof that can be easily digested and absorbed by infants. Source of starch and/or maltodextrin may be cereal flour or derivatives thereof, in particularly wheat, barley, rice and/or cornflour, and/or a starch, particularly wheat, barley, rice, tapioca, potato and/or corn starch, for example. Also glucose and/or fructose may be present.

For full term formulas prepared according to the invention, the carbohydrate source preferably comprises lactose, which may, at least partially, be provided by whole, preferably skimmed milk powder, for example.

For example, an infant formula prepared according to the invention may comprise, in percent by weight, 5-25%, preferably 10-20%, more preferably 12-18% of maltodextrin.

The digestible carbohydrate source may provide 50-70% of the energy of the formula, for example. Preferably the carbohydrate source provides 55-65%, more preferably 57-63%, for example 60% of the energy of the formula.

The lipid source may comprise any fat, oil or other lipid, which is suitable for use in nutritional formulas such as infant formulas, for example. Typical lipid sources include milk fat, safflower oil, egg yolk lipid, canola oil, olive oil, coconut oil, palm oil, palm kernel oil, palm olein, low eruic rapeseed oil, soybean oil, sunflower oil, fish oil, and microbial fermentation oil containing long-chain, polyunsaturated fatty acids. These oils may be in the form of high oleic forms such as high oleic sunflower oil and high oleic safflower oil. The lipid source may also be in the form of fractions derived from these oils such as palm olein, medium chain triglycerides (MCT), and esters of fatty acids such as arachidonic acid, linoleic acid, palmitic acid, stearic acid, docosahexaeonic acid, linolenic acid, oleic acid, lauric acid, capric acid, caprylic acid, caproic acid, and the like.

If the formula prepared according to the invention is intended for pre-term infants, the lipid source preferably contains medium chain triglycerides; for example in an amount of 10-40%, preferably 15-35% by weight of the lipid source.

The lipid source may provide 18-40% of the energy of the formula prepared according to the invention, for example. Preferably, the lipid source provides 20-35%, more preferably 25-30%, for example 27% of the energy of the formula.

An infant formula prepared according to the invention may further contain ingredients, which are designed to meet the nutritional needs of the human infant. In particular, it is preferred that the infant formula is "nutritionally complete"; that is it contains adequate nutrients to sustain healthy human life for extended periods. Preferably, the formula comprises vitamins, minerals and trace elements in recommended amounts.

For example, the formula prepared according to the invention may comprise magnesium, zinc, iron, copper, iodine, selenium, preferably in the form of biologically available salts. Furthermore, the formula may comprise vitamin C, A, E, B1, B2, B6, B12, D3, K1 (Phylloquinone) PP (Nicotinamide), Calcium-D-panthenate, D-Biotin, Taurin and nutritionally suitable mixtures thereof, for example.

In addition, the formula prepared according to the invention further comprises a food grade emulsifier, such as lecithin, for example.

The formula prepared according to the invention may further comprise further ingredients, for example with specific nutritional benefits or other desirable effects.

The process according to the present invention may comprise further steps, for example, after adding L(+)-lactic acid, adjusting the pH with a food-grade base and/or acid to between 3.5-6. Such a step may be useful to (fine-) adjust the pH more accurately to a selected value.

Since a powdered composition is to be produced, the homogenized and acidified solution comprising the carbohydrate, protein and lipid source is powdered by drying, for example spray-drying. In this case, minerals and/or vitamins may also be dry-added after drying.

Since L+lactic acid is added as indicated above (in-line), this has the advantage that the heat-treatment may be applied on a pH-neutral product. This reduces product fouling and simplifies the process in general, longer production runs are possible and cleaning of the material is easier.

A preferred process alternative for obtaining a powdered formula is given below in a more detailed manner.

Accordingly, the protein and carbohydrate source, for example skimmed milk powder, casein, whey and/or maltodextrin may be hydrated and dispersed in water to obtain a dispersion or solution with a dry matter content of 15-40%, preferably 20- 35%, more preferably 18-33%, most preferably 23-28% by weight. The solution is given time to allow for a proper hydration of the protein.

Alternatively, hydration may conveniently take place at lower contents of solids, for example if specific ingredients so require.

In a further step, minerals may be added to the solution. However, minerals and vitamins may equally be added, for example dry-mixed, at the end.

The lipid source, may be added at this moment. For example, it may be added after a pre-heating of the solution to 50 - 90°C, preferably 60 - 75°C. Thereby, the lipid source optionally including soy lecithin maybe added directly into a tank comprising the solution. Alternatively, lipids may be added in-line directly into the tube with the flowing solution.

The solution is preferably subjected to a heat treatment with the purpose of reducing bacterial load or facilitating homogenization or drying. For example, the solution may be heated to 95-120°C, preferably 100-110°C for 2 to 15 seconds, preferably 3 to 8 seconds, for example by direct steam injection with a Koreco steam injector.

L(+)-lactic acid is added in-line. The L(+)-lactic acid is diluted with water to obtain a solution.

Preferably, L+lactic acid may be added in-line to the heat-treated solution at about ambient temperatures, for example. For example, L+lactic acid may be diluted to 35-65%, preferably 40-60% by weight of L+lactic acid in water, and then added in-line. For example, L+lactic acid may be added in-line to the product-stream before homogenization.

In case L+-lactic acid is added in-line in the form of an about 50%-solution, it may be added in a rate of about 1.2-1.8 kg per 100 kg of the preferably heat-treated solution, preferably about 1.5 kg of diluted L+lactic acid per 100 kg of the solution, for example, depending on the acidity and pH to be obtained.

PH may be controlled by in-line measurement, for example, allowing for adjusting pH during the in-line adding by way of a feedback mechanism.

Preferably, pH is adjusted to 4.0 to 5.0, more preferably to 4.2 to 4.8, even more preferably to 4.3 to 4.6.

L(+)-lactic acid is commercially available, for example under the trademark PURAC® FCC 50 (PURAC biochem, Arkelsedijk 46, PO Box 21, 4200 AA Gorinchem, the Netherlands).

The amount of L(+)-lactic acid added depends on the exact pH or acidity wished to obtain. For example, based on the dry weight of the formula, it may comprise 0.5-3.5%, preferably 1-3%, more preferably 1.7-2.3% by weight of L(+)-lactic acid.

Preferably, a homogenisation step may be conducted before drying, in order to obtain an even, regular powder, for example. Hence, the concentrated solution may be preheated to 65-80°C, preferably 70-75°C and homogenised at a pressure of 100-200 bar, preferably 130-170bar, for example. While any suitable equipment may be used, an Alpha-Laval high pressure homogenizer is mentioned by way of example.

It may be useful to conduct an evaporation step before drying the solution in order to increase dry matter content. For example, the heated solution may be flashed into an evaporator, for example into a Scheffers or Niro falling-film evaporator in which the solution is concentrated up to 30 to 60%, preferably 45-55% dry matter.

Before spray drying, soy lecithin may be added to the solution, especially in case the lipid source has been added before acidification and without soy lecithin.

Spray drying may be conducted in order to obtain a powder suitable for reconstitution with water, for example. Other drying methods are possible and may be selected as well, such as fluidized bed drying, freeze drying, roller-drying for example.

If desired, additional ingredients may be added to the powder directly at the end and not to the solution, for example. This is particularly true for heat-sensitive ingredients, such as some vitamins, other beneficial metabolites, essential and non-essential molecules such as amino acids, such as taurine and L-carnitine, for example, and certain bio-active molecules, if not yet added to the solution, for example. Hence, the powder may be completed with a vitamin premix providing essential vitamins in amounts that are sufficient to cover basic requirements calculated on the base of the recommended daily consumption of the reconstituted formula.

The following examples are given by way of illustration only and in no way should be construed as limiting the subject matter of the present application. Percentages and parts are by weight unless otherwise indicated.

### Example 1: (not according to the invention)

A directly acidified, L-(+) lactic acid containing, powdered infant formula is prepared by using the ingredients of given in Table 1 below.

**Table 1: Ingredients for an Infant Formula in Percent**

| | |
|---|---|
| Dried whey powder | 43 |
| Maltodextrin¹ | 15 |
| Palm oil | 13 |
| Simmed milk powder | 5.4 |
| Rapeseed oil (low eruic) | 4.7 |
| Coconut oil | 4.4 |
| Potassium Caseinate | 4.4 |
| Sunflower oil | 2.7 |
| Lactic acid² | 2 |
| Soy lecithin (at 62%) | 1 |
| Water | 3 |

| | |
|---|---|
| ¹ Matlodextrin with D.E. (Dextrose equivalents) 24-32 was selected. ² L(+)-lactic acid was obtained from PURAC®, as an aqueous 50wt.-%-solution with a stereochemical purity of at least 95% (L-isomer). | |

Vitamins and minerals were added according to recommended values.

In order to prepare a powdered, reconstitutable infant formula, the maltodextrin, K-caseinate and skimmed milk powder are hydrated in tap water at about 50-60°C to obtain a solution. The solution is standardised to a total solids content (TS) of 25%. Hydration time was adapted to have a good hydration of the protein.

Some minerals (Ca-citrate, KCl, K-citrate, Na-citrate and MgCl₂) are added to the solution, which was then cooled down to 8°C.

L(+)-lactic acid is diluted in tap water at about 4°C to a concentration of about 10%.

L(+)-lactic acid is slowly added to the solution of hydrated ingredients at temperatures below 8°C.

The pH is adjusted with KOH and citric acid to a value between 4.3-4.4.

The solution is pre-heated to 50°C in a double-jacket oil tank, as well as the lipid source comprising palm oil, coconut oil, low eruic rarapeseed oil, sunflower oil and soy lecithin. The lipid source is then added in-line, that I, directly in the tube with the flowing product before the high-pressure homogenizer.

The solution including the added lipid source is heated to 105°C by direct steam injection by a steam injection valve and hold at the temperature for 5 seconds.

Then the product is directly flashed into an evaporator, in which the product is concentrated up to 40-50% total solids (dry matter) by a Scheffers falling-film evaporator.

Thereafter, the concentrated solution is conducted to a buffer tank for homogenisation, where it is pre-heated to 75°C, homogenised at 150 bars with a high pressure pump and then spray dried.

The powdered solution is then supplied with the vitamin premix, the mineral premix and a small part of the maltodextrin.

The powder obtained is a formula particularly suitable for infants that may be reconstituted with tap water (20 g powder with 137 ml water, for example). The recommended daily serving size for a 3 month old baby would be 153 g powder and 900 ml water.

The pH of the reconstituted formula is 4.5.

### Example 2: Alternative preparation of an acidified nutritional formula (not according to the invention)

A ready-to-drink formula was prepared based on the ingredients, percentages and the procedure according to Example 1, with the exceptions that no soy lecithin is present. Furthermore, the process is conducted at a dry-matter content in the range of 12-14% by weight and an UH-treatment is conducted at the end, before aseptically filling into packs of 240 ml:
- mixing and hydrating carbohydrates and proteins,
- pre-heating the solution to about 50°C,
- pre-heating the lipid source to about 50°C, adding a lipid source (in line).
- homogenising the solution,
- adding L-(+) lactic acid to obtain a pH between 4.3-4.4,
- UHT-treating the solution for sterilisation,
- aseptically filling the dispersion into packs of 240 ml.

A ready to drink formula with a dry-matter content of 12.5% is obtained.

### Example 3: Preparation according to the invention of an acidified nutritional formula

A nutritional composition was prepared with the basic ingredients given in Table 2 below.

**Table 2: Ingredients / raw materials for nutritional composition.**

| | % raw material | % dry matter of the ingredient | Kg raw material |
|---|---|---|---|
| Skimmed milk | 37.8 | 97 | 584.1 |
| Malto-dextrin 24-32 | 15.5 | 96 | 242.6 |
| Sucrose | 11.1 | 100 | 166.5 |
| native corn starch | 9.0 | 88 | 153.4 |
| Corn oil | 2.5 | 100 | 38 |
| Rapeseed oil | 4.0 | 100 | 60.0 |
| Palm olein DF | 10.8 | 100 | 161.6 |
| Palm kernel oil | 3.8 | 100 | 57.0 |
| Lecithin soya at 62% | 0.5 | 100 | 7.50 |
| L+-Lactic acid | 2.0 | 50 | 60.0 |

Skimmed milk, maltodextrin, sucrose and native starch are dissolved in water at about 50°C at a dry-matter content of 31% by weight.

Fat is added at the same temperature, and dry matter content is adjusted to 30% by weight.

The solution is heated to 70°C with a plate heater, then a heat treatment to reduce bacterial load is conducted at 110°C for 10s by direct steam injection.

The solution having dry matter content of 36% and 70°C is flushed into a buffer tank. From there, the solution is led to the homogeniser.

L+ lactic acid is added at 25°C in the form of a 50% solution in-line into the tube leading to the homogeniser. PH is measured in-line and L+ lactic acid is added in amounts as to obtain a pH of 5.0. The L+ lactic acid solution is added at a rate of 1.5 kg per 100 kg of the hydrated and heat-treated solution.

A two-stage homogenisation is conducted at 100 bar and 20 bar, respectively, and dry matter is preferably at about 36% at this moment.

Thereafter, the homogenised solution is spray-dried and a powdered nutritional composition is obtained. The powdered composition was completed with sufficient amounts of minerals and vitamins and filled into cans.

The powdered composition was reconstituted (13g powder in 90 ml water), and a reconstituted composition is obtained, having a pH in the range of 4.9-5.1.

The powdered composition obtained has essentially the same product characteristics as the powderes obtained according to Examples 1 - 2. However, the process according to the present example is more efficient and allows for higher turnover.

### Example 4 : Microbiological Tests

The infant formula of Example 1 and different commercially available infant formulas were subjected to microbiological tests with various gastrointestinal pathogens. Table 1 lists the different formulas and indicates the pH that is obtained after reconstitution.

**Table 1: Comparison of different infant formulas**

| **Product** | **Main Characteristics** | **pH** |
|---|---|---|
| A | Whey-adapted | 6.8 |
| B | Casein-predominant | 6.8 |
| C | Soy-based | 6.9 |
| D | Whey-adapted, fermented | 4.7 |
| E | Directly acidified | 4.5 |
| F | Directly acidified | 5.0 |
| G | Directly acidified | 5.2 |

### MICROBIAL CULTURES

*Escherichia coli* O157:H7, *Pseudomonas aeruginosa, Staphylococcus aureus, Bacillus cereus, Salmonella typhimurium, Shigella dysenteriae* FSM 1, 2 and 3, *Enterobacter sakazakii, Vibrio cholerae* O:139, *Candida albicans* and Rotavirus WA (Human Rotavirus serotype 1), Hochi (Human Rotavirus serotype 4) and SA11 (Simian Rotavirus corresponding to Human Rotavirus serotype 3) were all from the strain collection of the Nestle Research Centre. Numbers behind the species name indicate serotype of the strain. These strains are most likely representative for other pathogenic strains of the same species. Any pathogen from these species, which is not particularly selected or genetically modified, would probably behave accordingly in the below-given experimental setting.

### CHALLENGE TEST

Bacterial strains were grown individually in Brain Heart Infusion (BHI, Oxoid CM225) for 18 to 20 h at 37°C. After dilution with Tryptone Salt (0.1 % tryptone (Oxoid LR42 + 0.85% NaCl) to a concentration of ca. 10⁵ CFU/ml, suspensions of strains belonging to the same species were pooled and aliquots of 1 ml were subsequently added to bottles containing 100 ml of the freshly prepared reconstituted infant formula thus yielding an initial concentration of ca 10³ cfu/ml. For *Candida* the same procedure was followed except that it was grown in Yeast and Malt extract broth (YM, Difco 0711-17-1).

After inoculation the bottles were incubated in a water bath at 4, 25 or 37°C and growth or inactivation was monitored by enumeration after 0, 3 and 6 hours. For this purpose the following media were employed: Violet Red Bile Glucose agar (VRBG, Oxoid CM 485) for the *Enterobacteriaceae,* MYP (Merck 5267) for *B. cereus,* Baird-Parker Agar (Oxoid CM 275) for *S. aureus,* Cholera Medium (TCBS, Oxoid CM 333) for *V. cholerae,* KF Streptococcus agar (Oxoid CM 701) for *Ent. faecalis,* DG18 (Oxoid CM729) for *C*. *albicans* and Pseudomonas Agar (Oxoid CM559) for *Ps. aeruginosa.* In order to determine the reproducibility of the results some of the challenge tests were performed in triplicate.

### RESULTS AND DISCUSSION

### MICROBIOLOGICAL QUALITY

To verify that the starting material itself was not contaminated several microbiological tests were performed. The results (data not shown) demonstrate that all the infant formulas were free (< 1 cfu/ml of reconstituted product) of *Enterobacteriacae, Ps. aeruginosa, B. cereus, S. aureus,* Enterococci, yeasts and moulds. Total plate counts were less than 50 cfu/ml.

### FATE OF MICROBIAL PATHOGENS IN RECONSTITUTED INFANT FORMULA

In the first challenge test, four commercially available infant formulas were separately inoculated with eight different bacterial pathogens and one spoilage yeast. The temperature was found to have a major effect on the behaviour of the various pathogens. At 4°C the tested pathogens apparently did not grow nor die in any of the products during the 6h following reconstitution. At 25°C, most bacteria showed some (albeit slow) growth in the pH-neutral products, but in the acidified product "D" no growth occurred. The yeast *C*. *albicans* did not grow nor die off in any of the products.

At 37°C, all bacteria grew quite well in the pH-neutral products, but no or only very little growth occurred in the infant formula with a low pH. For *Ps. aeruginosa* exposure to the acidified product at 37°C had a clear bactericidal effect. *C. albicans* counts again showed little variation.

As these results showed that the fermented product had a clear bacteriostatic effect on pathogenic bacteria the question was raised whether this was solely due to the presence of lactic acid and the reduced pH or whether other inhibitory factors were involved. To address this a second challenge test was performed in which the antimicrobial properties of the fermented product ("D") were compared with those of a directly acidified non-fermented product ("E"), the formula obtained in Example 1.

It was found that for the fate of the pathogens, it did not make any difference whether they were exposed to the non-fermented acidified product or to the fermented product. The *Enterobacteriaceae (S. typhimurium, Sh. dysenteriae* and *E. coli O157:H7)* resisted fairly well under the conditions tested (6 hours at 25 and 37°C), but for *V*. *cholerae* the exposure to this environment quickly became lethal at both temperatures. Already within three hours it was no longer detectable.

In the same experiment the influence of the pH was further investigated; it was found that already at pH 5.0 *Salmonella* and *E*. *coli* O157 were no longer inhibited, whereas *Shigella* started to grow at pH 5.2. For *Vibrio,* on the other hand, this pH was still bactericidal.

This example study shows that pH-neutral infant formulas may support rapid growth of enteric pathogens when stored at 25 or 37°C after reconstitution. To prevent the risk that such products become hazardous, they should only be stored for a short time or under refrigeration. For acidified formulas, the most relevant pathogens cannot grow at pH of 5.0 or lower. Products which have been acidified through fermentation with lactic acid bacteria have similar bacteriostatic properties as formulas which have been acidified through direct addition of lactic acid and both may provide a safe alternative for the feeding of infants in situations where breast-feeding may not be possible.

## Claims

1. A method of preparing a powdered nutritional formula comprising a protein source, a carbohydrate source and a lipid source, wherein the pH of the formula, in its liquid state, is in the range of 3.5 to 6, wherein the formula comprises lactic acid and whereby at least 70% by weight of the lactic acid is present as the enantiomer of L(+)-lactic acid, comprising the steps of
- hydrating the protein source and the carbohydrate source;
- adding the lipid source;
- adding diluted L-(+) lactic acid to the hydrated carbohydrate source, the hydrated protein source and the lipid source until a pH of about 3.5 - 6 is obtained, the lactic acid addition taking place in-line; and
- drying the obtained mixture.

## Patentansprüche

1. Verfahren zur Herstellung einer pulverförmigen Ernährungs-Formulanahrung, die eine Proteinquelle, eine Kohlenhydratquelle und eine Lipidquelle umfasst, wobei der pH-Wert der Formulanahrung in ihrem flüssigen Zustand im Bereich von 3,5 bis 6 liegt, wobei die Formulanahrung Milchsäure umfasst und wobei mindestens 70 Gew.-% der Milchsäure als das Enantiomer der L(+)-Milchsäure vorliegen, umfassend die Schritte
- Hydratisieren der Proteinquelle und der Kohlenhydratquelle;
- Zugeben der Lipidquelle;
- Zugeben von verdünnter L(+)-Milchsäure zu der hydratisierten Kohlenhydratquelle, der hydratisierten Proteinquelle und der Lipidquelle, bis ein pH-Wert von etwa 3,5-6 erreicht ist, wobei die Milchsäurezugabe inline erfolgt; und
- Trocknen der erhaltenen Mischung.

## Revendications

1. Procédé de préparation d'une formule nutritionnelle en poudre comprenant une source de protéine, une source de glucide et une source de lipide, dans lequel le pH de la formule, à l'état liquide, est compris dans la plage de 3,5 à 6, dans lequel la préparation comprend de l'acide lactique et dans lequel au moins 70% en poids de l'acide lactique est présent sous forme de l'énantiomère acide lactique L(+), comprenant les étapes suivantes :
- hydratation de la source de protéine et de la source de glucide ;
- ajout de la source de lipide ;
- ajout d'acide lactique L(+) dilué à la source de glucide hydratée, à la source de protéine hydratée et à la source de lipide jusqu'à l'obtention d'un pH d'environ 3,5 à 6, l'ajout d'acide lactique étant réalisé en ligne ; et
- séchage du mélange obtenu.
